# FASCICULE DE BREVET EUROPEEN

(11) **EP 1 025 819 B1**
(45) Date de publication et mention de la délivrance du brevet: **03.07.2013**
(21) Numéro de dépôt: 00400211.9
(22) Date de dépôt: 27.01.2000
(51) Int. Cl.: A61F 2/38

(54) **Prothese totale du genou à insert mobile par rapport à un tenon**
Knieprothese mit relativ zu einem Zapfen bewegbarem Einsatz
Knee prosthesis with an insert, which is mobile in relation to a post

(30) Priorité: 02.02.1999 FR 9901158; 05.07.1999 FR 9908632
(43) Date de publication de la demande: 09.08.2000
(73) Titulaire: Aesculap AG, 78532 Tuttlingen (DE)
(72) Inventeur: Marceaux, Pascal, 52000 Chaumont (FR); Biegun, Jean-François, 52000 Chaumont (FR); Jenny, Jean-yves, 67460 Lipsheim (FR); Miehlke, Rolf, 48167 Münster (DE); Saragaglia, Dominique, 38640 Claix (FR)
(74) Mandataire: Eidelsberg, Victor Albert

(56) Documents cités:
- EP-A- 0 529 408
- EP-A- 0 546 726
- WO-A-99/13804
- FR-A- 2 758 456
- US-A- 5 609 639

## Description

La présente invention concerne une prothèse totale d'articulation du genou, constituée d'une partie tibiale, d'une partie fémorale et d'un insert tibial assurant la liaison entre la partie tibiale et la partie fémorale. La présente invention se rapporte également à un insert tibial de ce genre.

On connaît déjà une prothèse totale d'articulation du genou de ce genre de la demande de brevet européenne N° 92 113726.1, au nom de la Société AESCULAP AG. Suivant ce document de l'art antérieur, la prothèse totale du genou est constituée d'une partie tibiale, comportant une plaque tibiale fixée à la partie proximale du tibia par l'intermédiaire d'une tige d'ancrage ancrée dans la cavité médullaire du tibia, d'une partie fémorale, comportant deux condyles, fixée à la partie distale du fémur, les condyles ayant des surfaces extérieures de forme sphérique, et d'un insert tibial, disposé sur la plaque tibiale et ayant sur sa partie supérieure des surfaces de forme complémentaire des surfaces des condyles pour permettre un glissement des condyles par rapport à cet insert tibial, pour permettre une flexion relative de la partie tibiale et de la partie fémorale. En outre, il est prévu un organe de couplage de l'insert tibial avec la plaque tibiale, constitué d'un tourillon monté en pivot dans un alésage ménagé au centre de la plaque tibiale et d'un appendice qui coopère avec une rainure formée dans la partie inférieure de l'insert tibial pour un guidage en translation et en rotation dans le plan de la plaque tibiale de l'insert tibial par rapport à cette plaque tibiale.

Cette prothèse totale du genou présente l'inconvénient que lors de la pose de la prothèse, le praticien doit centrer parfaitement celle-ci en position pour correspondre exactement aux anatomies du fémur et du tibia. La moindre erreur de centrage oblige le praticien à recommencer.

Aux documents FR-A-2758456 et US-A-5609639, il est décrit une prothèse suivant le préambule de la revendication 1.

Suivant l'invention une prothèse totale d'articulation du genou est tel que définie à la revendication 1, les sous revendications visant des perfectionnements. Un insert tibial selon l'invention est défini à la revendication 13.

Ainsi le centrage de la prothèse s'effectue de manière dynamique par simple déplacement médio-latéral de l'insert par rapport à la plaque tibiale, lors de la pose de la prothèse.

Les prothèses totales du genou de l'art antérieur présente en outre l'inconvénient que, lors de la flexion du genou, et en raison de mouvements incontrôlés, le tourillon peut se déloger de l'alésage dans lequel il est monté dans la plaque tibiale, et entraîner ainsi une séparation non souhaitable de l'insert tibial et de la partie fémorale par rapport à la plaque tibiale.

En outre, la rotation de l'insert tibial par rapport à la plaque tibiale n'est pas limitée, puisque l'organe de couplage peut effectuer une rotation complète autour de l'alésage, ce qui ne correspond pas à la rotation habituellement observée dans le cas du genou anatomique.

Suivant un perfectionnement de l'invention, le tenon est monté fixe sur la plaque tibiale.

En prévoyant ainsi de fixer le tenon à la plaque tibiale, on s'assure en outre de l'impossibilité que ce même tenon puisse se déloger de l'alésage formé dans la plaque tibiale. Pour permettre cependant une rotation de l'insert tibial par rapport à la plaque tibiale, on prévoit une cavité de dimension suffisante pour permettre une rotation de la partie oblongue du tenon dans la cavité sur au moins un domaine de rotation donné, par exemple de ± 25°, de préférence ± 15°.

Suivant un mode de réalisation préféré, on prévoit que la partie oblongue est à distance de la plaque tibiale et que la cavité comprend une première ouverture latérale ménagée dans la paroi latérale antérieure de l'insert tibial et une deuxième ouverture de fond, la dimension de l'ouverture latérale permettant le passage de la partie oblongue à travers elle et l'ouverture de fond étant délimitée par des bords inférieurs des parois latérales de la cavité et par un bord inférieur d'un rebord inférieur défini par la première ouverture latérale de la butée de manière à former un trou de limitation qui limite le déplacement en glissement en translation antéro-postérieur et médio-latéral de la base du tenon par rapport à la plaque tibiale, le rebord inférieur de la première ouverture formant une butée qui coopère avec la partie oblongue de manière à empêcher un détachement par le haut de l'insert tibial de la plaque tibiale.

Le fait de prévoir cette butée permet, dans le cas où l'on coupe le ligament croisé postérieur (LCP) d'éviter la luxation antérieure de l'insert par rapport au tibia, la butée venant remplacer le LCP dans sa fonction de limitation du déplacement antérieur lors de la flexion du genou.

L'invention vise également un insert tibial tel qu'utilisé dans la prothèse tibiale du genou décrite précédemment.

On décrit maintenant à titre d'exemple un mode de réalisation d'une prothèse totale de genou à insert mobile suivant l'invention, en se référant au dessin, donné uniquement à titre d'exemple, dans lequel :
la figure 1 est une vue en perspective de dessus de l'insert tibial et d'une plaque tibiale suivant l'invention, qui sont à l'état non assemblé.
La figure 2 est une vue en perspective de dessous de l'insert tibial et de la plaque tibiale de la figure 1.
Les figures 3A, 3B et 3C sont des vues de dessus montrant les déplacements mutuels possibles de l'insert tibial et de la plaque tibiale, à l'état assemblé de l'insert tibial sur la plaque tibiale.
La figure 4 est une vue en coupe suivant la ligne A-A de la figure 3A.
La figure 5 est une vue en perspective de dessus de l'insert tibial et d'une plaque tibiale qui sont à l'état non assemblé d'un mode de réalisation en variante de l'invention.
La figure 6 est une vue en perspective de dessous de l'insert tibial et de la plaque tibiale de la figure 5.
Les figures 7A, 7B, 7C et 7D sont des vues de dessus montrant les déplacements mutuels possibles de l'insert tibial et de la plaque tibiale, à l'état assemblé de l'insert tibial sur la plaque tibiale des figures 5 et 6.
La figure 8 est une vue en coupe suivant la ligne A-A de la figure 7A.

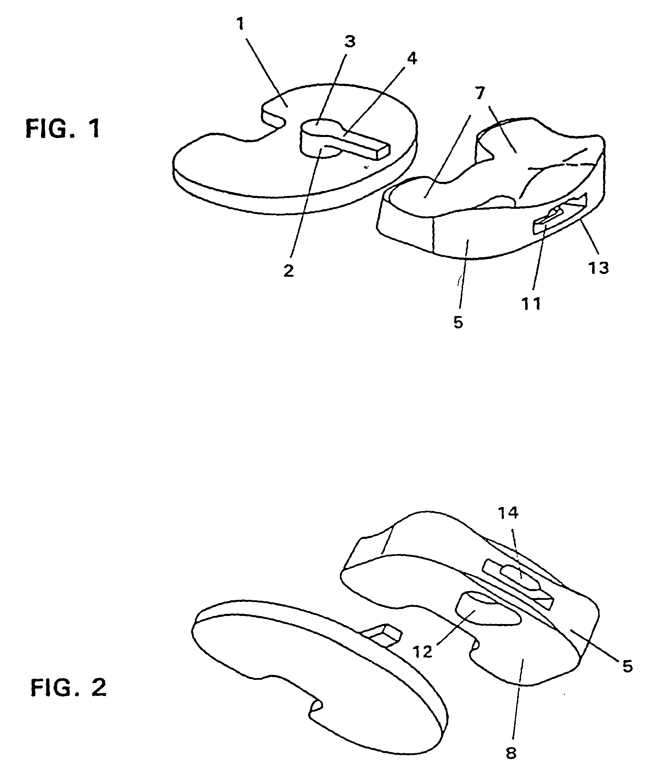

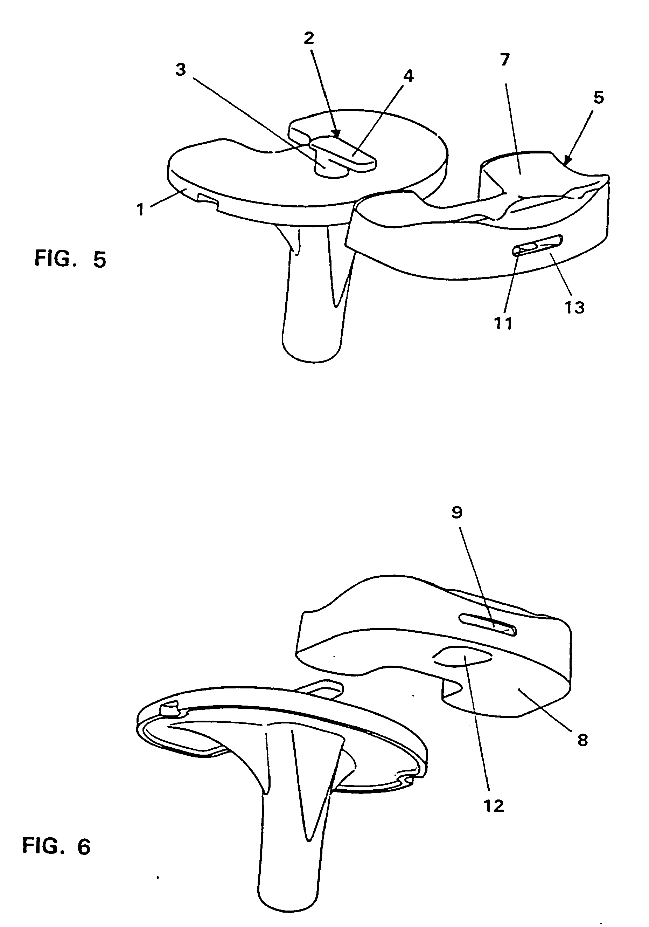

Le mode de réalisation décrit aux figures 5 à 8 est similaire à celui des figures 1 à 4. Les mêmes éléments ou les éléments ayant la même fonction y sont désignés par les mêmes références numériques.

A la figure 1, il est représenté une plaque tibiale 1 destinée à être ancrée à l'extrémité proximale d'un tibia (non représenté) par l'intermédiaire d'une pièce d'ancrage (non représentée également) disposée sous la plaque tibiale. De cette plaque tibiale 1, fait saillie de sa partie supérieure un tenon 2, constitué d'une base 3 et d'un prolongement 4 en forme de barrette de section transversale rectangulaire. La base 3 du tenon est fixée à la plaque tibiale 1 de manière non amovible et sans possibilité de rotation, et le prolongement 4 est orienté en ayant sa longueur dirigée dans le sens antéro-postérieur (la direction antéro-postérieur étant la direction allant du creux du genou vers la partie avant du genou, où le genou fait saillie). La section droite transversale du prolongement 4 est de forme rectangulaire, mais cette forme pourrait également être circulaire, elliptique, carrée ou autre. Le prolongement 4 s'étend à partir de la partie supérieure de la base 3 du tenon en étant à distance de la plaque tibiale (de 1 mm à 10 mm).

Suivant un autre mode de réalisation possible de l'invention, on peut prévoir que le prolongement 4 du tenon 2 soit directement en contact avec la plaque tibiale 1, et de ne pas prévoir la butée décrite ci-après dans la cavité de l'insert tibial (voir la description plus loin) ; ce second mode de réalisation possible permettrait également l'obtention d'un certain nombre d'avantages de l'invention à savoir la limitation de la dislocation par empêchement de dislocation du tenon 2 de la plaque tibiale, la tolérance d'un déplacement médio-latéral et donc l'obtention d'un bon centrage du fémur par rapport au tibia et la limitation de la rotation mutuelle de l'insert tibial par rapport à la plaque tibiale.

A l'état monté, la plaque tibiale 1 reçoit sur sa surface supérieure un insert tibial 5, l'insert tibial 5 étant par exemple en un matériau polyéthylène ou analogue, et ayant une face supérieure constituée de deux surfaces 7 incurvées dont la concavité est tournée vers le haut et qui sont destinées à recevoir chacune une surface incurvée de manière complémentaire d'un condyle respectif de deux condyles (non représentés) fixés à l'extrémité distale du fémur. L'insert tibial 5 comprend également une surface inférieure plate 8 qui est destinée, en position montée de la prothèse totale du genou, à reposer sur la plaque tibiale 1 et à y glisser.

Il est prévu dans l'insert tibial 5 une cavité 9. Cette cavité 9 est formée dans la partie inférieure de l'insert tibial 5, dans la partie antérieure. Cette cavité 9 est symétrique par rapport au plan médio-latéral de l'insert tibial, c'est-à-dire le plan comprenant d'une part l'axe antéro-postérieur et d'autre part l'axe normal à la surface inférieure de l'insert tibial. La cavité 9 est délimitée dans sa partie supérieure par une paroi 10 supérieure, et latéralement par une première et une deuxième parois latérales 15, 16 qui forment entre elles un angle approximativement de 30° et qui se réunissent dans la partie postérieure de l'insert tibial suivant une forme arrondie correspondant à la forme arrondie ou circulaire de la paroi latérale de la base 3 du tenon 2.

En outre, la cavité 9 comprend une première ouverture 11 latérale et une deuxième ouverture 12 de fond. L'ouverture 12 de fond a une forme de triangle isocèle, notamment équilatéral, dont les côtés se rejoignent par des arrondis de forme complémentaire à l'arrondi de la base 3 du tenon 2. La première ouverture 11 est formée dans la partie latérale antérieure de l'insert tibial 5 et a des dimensions suffisantes pour y permettre le passage du prolongement 4, ainsi que le débattement en rotation de ce même prolongement 4 d'une paroi latérale 15, 16 à l'autre de la cavité 9.

Suivant le mode de réalisation décrit ici, il est prévu, entre les deux ouvertures 11 et 12, une butée 13 constituée du bord inférieur antérieur de l'insert tibial 5 et qui, en position montée de la prothèse totale du genou, vient se positionner sur la plaque tibiale 1, sous le prolongement 4, en empêchant ainsi un détachement trop facile de l'insert tibial de la plaque tibiale, ou une dislocation de l'insert tibial de la plaque.

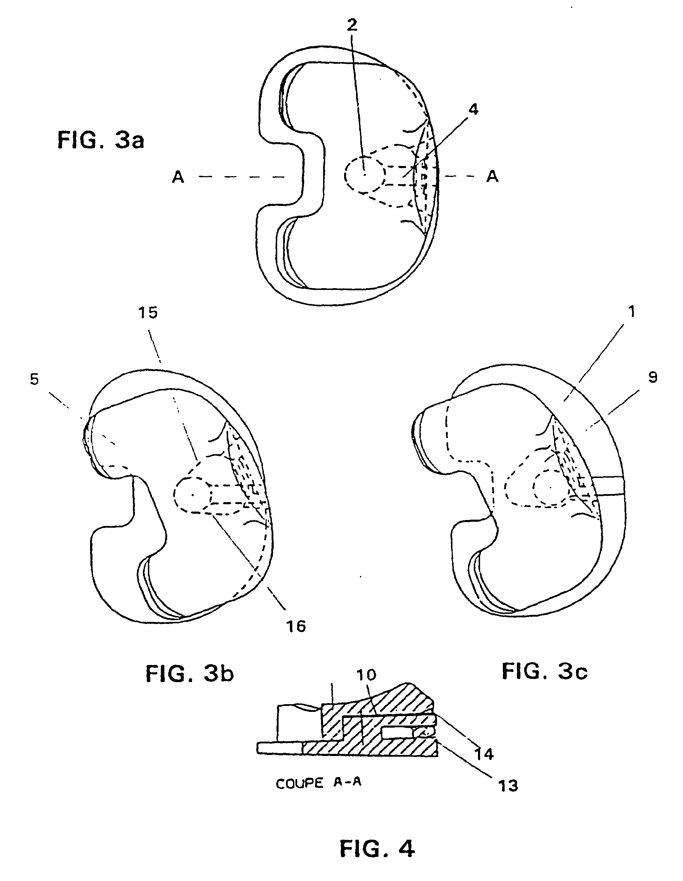

Les figures 3A, 3B, 3C montrent les déplacements mutuels de l'insert 5 tibial par rapport à la plaque 1 tibiale. A la figure 3A, la plaque tibiale et l'insert tibial sont orientés parallèlement l'un à l'autre, la cavité 9 étant divisée en deux partie égales par la base 3 et le prolongement 4 du tenon 2. L'insert tibial peut tourner par rapport à la plaque tibiale, ce déplacement en rotation étant possible jusqu'à ce que le prolongement 4 vienne buter contre la paroi 16 latérale de la cavité. On obtient ainsi une rotation d'environ plus 15° de l'insert tibial par rapport à la plaque tibiale. Une rotation équivalente dans l'autre sens est également possible de sorte que le débattement de la plaque tibiale par rapport à l'insert tibial est d'environ plus ou moins 15°. En modifiant l'angle entre les parois latérales 15, 16, on peut régler la limitation de la rotation mutuelle de l'insert et de la plaque tibiale. En général, on choisira un angle compris entre 30° et 50°, ce qui correspond à une limitation de la rotation mutuelle comprise entre ± 15 et ± 25°. En outre, un déplacement en translation du tenon suivant l'axe antéro-postérieur du genou est possible, comme représenté à la figure 3C. Ce déplacement est limité par la butée 13 sur laquelle vient buter la base 3 du tenon 2 après une course vers l'arrière de l'insert tibial par rapport à la plaque tibiale.

Dans l'autre sens (course vers l'avant de l'insert tibial par rapport à la plaque tibiale), la base 3 du tenon 2 bute également contre le coin commun aux deux parois 15 et 16. Cette limitation du déplacement relatif de l'insert et de la plaque permet d'utiliser la prothèse du genou en conservant le Ligament Croisé Postérieur (LCP) ou en le coupant. Dans ce dernier cas, c'est le coin commun des deux parois 15, 16 qui assure la limitation du déplacement antéro-postérieur, en remplacement du LCP, ce qui permet d'éviter une luxation de l'insert lors de la flexion du genou.

En outre, on peut voir qu'un déplacement médio-latéral de la plaque tibiale par rapport à l'insert tibial est possible, lorsque la base 3 du tenon 2 se déplace le long des parois latérales 15, 16 de la cavité ou le long de la face intérieure de la butée 13 ou à l'intérieur du domaine défini par ces parois latérales et cette butée 13. On obtient ainsi une compensation dynamique du centrage de la partie fémorale par rapport à la partie tibiale, qu'il est parfois difficile de réaliser de manière statique lors de la pose de la prothèse.

Suivant un mode de réalisation supplémentaire possible de l'invention, on peut prévoir au niveau de la partie supérieure de l'ouverture latérale de la cavité 9, une découpe 14 qui facilite l'introduction du tenon 2 dans la cavité 9 et à travers l'ouverture 11 lors de la mise en place de la prothèse du genou.

A la figure 5, il est représenté une plaque tibiale 1 destinée à être ancrée à l'extrémité proximale d'un tibia (non représenté) par l'intermédiaire d'une pièce d'ancrage disposée sous la plaque tibiale. De cette plaque tibiale 1, fait saillie de sa partie supérieure un tenon 2, constitué d'une base 3 et d'un prolongement 4 en forme de barrette biseautée de section transversale rectangulaire. La base 3 du tenon est fixée à la plaque tibiale 1 de manière non amovible et sans possibilité de rotation, et le prolongement 4 est orienté en ayant sa longueur dirigée dans le sens antéro-postérieur (la direction antéro-postérieur étant la direction allant du creux du genou vers la partie avant du genou, où le genou fait saillie). La section droite transversale du prolongement 4 est de forme rectangulaire, mais cette forme pourrait également être circulaire, elliptique, carrée ou autre. Le prolongement 4 s'étend à partir de la partie supérieure de la base 3 du tenon en étant à distance de la plaque tibiale (de 1 mm à 10 mm).

Suivant un autre mode de réalisation possible de l'invention, on peut prévoir que le prolongement 4 du tenon 2 soit directement en contact avec la plaque tibiale 1, et de ne pas prévoir la butée décrite ci-après dans la cavité de l'insert tibial (voir la description plus loin)

A l'état monté, la plaque tibiale 1 reçoit sur sa surface supérieure un insert tibial 5, l'insert tibial 5 étant par exemple en un matériau polyéthylène ou analogue, et ayant une face supérieure constituée de deux surfaces 7 incurvées dont la concavité est tournée vers le haut et qui sont destinées à recevoir chacune une surface incurvée de manière complémentaire d'un condyle respectif de deux condyles (non représentés) fixés à l'extrémité distale du fémur. L'insert tibial 5 comprend également une surface inférieure plate 8 qui est destinée, en position montée de la prothèse totale du genou, à reposer sur la plaque tibiale 1 et à y glisser.

Il est prévu dans l'insert tibial 5 une cavité 9. Cette cavité 9 est formée dans la partie inférieure de l'insert tibial 5, dans la partie antérieure. Cette cavité 9 est symétrique par rapport au plan médial de l'insert tibial, c'est-à-dire le plan comprenant d'une part l'axe antéro-postérieur et d'autre part l'axe normal à la surface inférieure de l'insert tibial. La cavité 9 est délimitée dans sa partie supérieure par une paroi 10 supérieure, et latéralement par une première et une deuxième parois latérales 15, 16 qui forment entre elles un angle approximativement de 30° et qui se réunissent dans la partie postérieure de l'insert tibial suivant une forme arrondie correspondant à la forme arrondie ou circulaire de la paroi latérale de la base 3 du tenon 2.

En outre, la cavité 9 comprend une première ouverture 11 latérale et une deuxième ouverture 12 de fond. L'ouverture 12 de fond a une forme de triangle isocèle, notamment équilatéral, dont les côtés se rejoignent par des arrondis de forme complémentaire à l'arrondi de la base 3 du tenon 2. La première ouverture 11 est formée dans la partie latérale antérieure de l'insert tibial 5 et a des dimensions suffisantes pour y permettre le passage du prolongement 4, ainsi que le débattement en rotation de ce même prolongement 4 d'une paroi latérale 15, 16 à l'autre de la cavité 9.

Suivant le mode de réalisation décrit ici, il est formé, entre les deux ouvertures 11 et 12, un rebord formant butée 13 constituée du bord inférieur antérieur de l'insert tibial 5 et qui, en position montée de la prothèse totale du genou, vient se positionner sur la plaque tibiale 1, sous le prolongement 4, en empêchant ainsi un détachement trop facile de l'insert tibial de la plaque tibiale, ou une dislocation de l'insert tibial de la plaque.

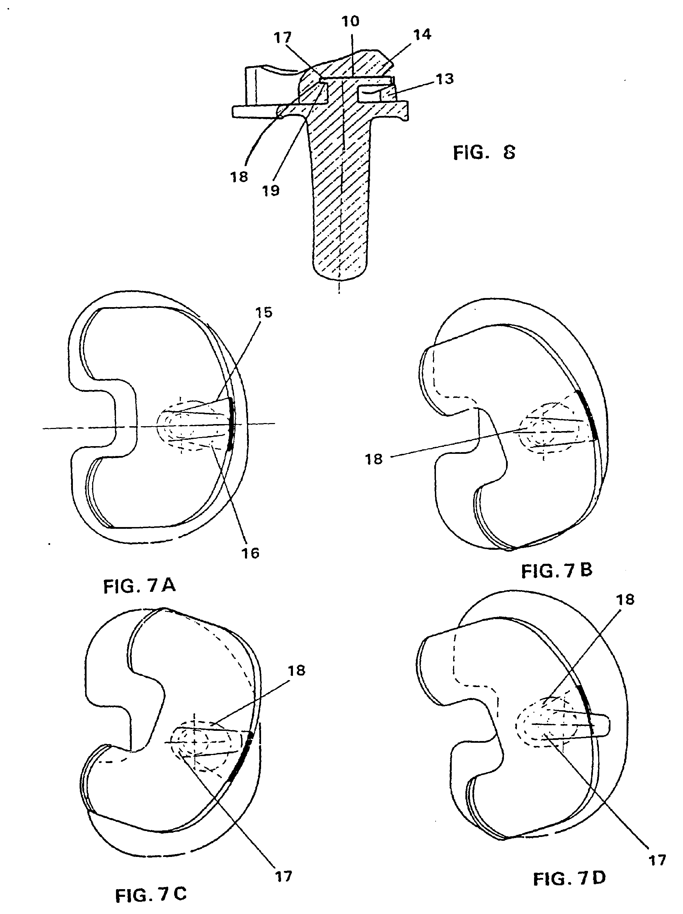

Les figures 7A, 7B, 7C montrent les déplacements mutuels de l'insert 5 tibial par rapport à la plaque 1 tibiale. A la figure 7A, la plaque tibiale et l'insert tibial sont orientés parallèlement l'un à l'autre, la cavité 9 étant divisée en deux parties égales par la base 3 et le prolongement 4 du tenon 2. L'insert tibial peut tourner par rapport à la plaque tibiale, ce déplacement en rotation étant possible jusqu'à ce que le prolongement 4 vienne buter contre la paroi 16 latérale de la cavité (figure 7B). On obtient ainsi une rotation d'environ plus 15° de l'insert tibial par rapport à la plaque tibiale. Une rotation équivalente dans l'autre sens est également possible (figure 7C) de sorte que le débattement de la plaque tibiale par rapport à l'insert tibial est d'environ plus ou moins 15°. En modifiant l'angle entre les parois latérales 15, 16, on peut régler la limitation de la rotation mutuelle de l'insert et de la plaque tibiale. En général, on choisira un angle compris entre 30° et 50°, ce qui correspond à une limitation de la rotation mutuelle comprise entre ± 15 et ± 25°. En outre, un déplacement en translation du tenon suivant l'axe antéro-postérieur du genou est possible, comme représenté à la figure 7D. Ce déplacement est limité par la butée 13 sur laquelle vient buter la base 3 du tenon 2 après une course vers l'arrière de l'insert tibial par rapport à la plaque tibiale.

Dans l'autre sens (course vers l'avant de l'insert tibial par rapport à la plaque tibiale), la base 3 du tenon 2 bute également contre le coin commun aux deux parois 15 et 16. Cette limitation du déplacement relatif de l'insert et de la plaque permet d'utiliser la prothèse du genou en conservant le Ligament Croisé Postérieur (LCP) ou en le coupant. Dans ce dernier cas, c'est le coin commun des deux parois 15, 16 qui assure la limitation du déplacement antéro-postérieur, en remplacement du LCP, ce qui permet d'éviter une luxation de l'insert lors de la flexion du genou.

En outre, on peut voir qu'un déplacement médio-latéral de la plaque tibiale par rapport à l'insert tibial est possible, lorsque la base 3 du tenon 2 se déplace le long des parois latérales 15, 16 de la cavité ou le long de la face intérieure de la butée 13 ou à l'intérieur du domaine défini par ces parois latérales et cette butée 13. On obtient ainsi une compensation dynamique du centrage de la partie fémorale par rapport à la partie tibiale, qu'il est parfois difficile de réaliser de manière statique lors de la pose de la prothèse.

Suivant un mode de réalisation supplémentaire possible de l'invention, on peut prévoir au niveau de la partie supérieure de l'ouverture latérale de la cavité 9, une découpe 14 qui facilite l'introduction du tenon 2 dans la cavité 9 et à travers l'ouverture 11 lors de la mise en place de la prothèse du genou.

La partie 4 oblongue fait en outre saillie dans l'autre sens, opposé au sens dans lequel elle s'étend de la base 3, par une protubérance 17 en forme de demi disque. Cette protubérance 17 coopère avec une rainure 18 formée dans la cavité 9. Dans le plan médial, c'est-à-dire dans le plan défini par l'axe antéro-postérieur et l'axe longitudinal du tibia (normale au plan de la plaque tibiale), les formes de la protubérance 17 et de la rainure 18 sont complémentaires. Cette complémentarité de forme permet un blocage de la protubérance 17 dans la rainure 18, par l'intermédiaire du bord 19 inférieur de la rainure 18 qui fait office de butée pour la protubérance, vis-à-vis d'un basculement par rapport à un axe perpendiculaire au plan de symétrie, dans le sens gauche droite, c'est-à-dire empêche que l'insert 5 ne se détache de la plaque tibiale 1. Cet effet anti-basculement est peut être prévu seul, c'est-à-dire sans prévoir la butée 13 (par exemple, comme indiqué précédemment lorsque le prolongement 4 est directement en contact avec la plaque tibiale) qui a également pour fonction, en coopération avec la partie 4 oblongue, d'empêcher un basculement de ce genre, ou en combinaison avec cette butée 13 pour un effet anti-basculement encore plus important, et notamment dans les deux sens, c'est-à-dire un basculement de l'insert par rapport à la plaque tibiale dans le sens des aiguilles d'une montre (butée 19) à la figure 4 ou dans le sens inverse (butée 13).

La dimension de la rainure 18, dans le plan perpendiculaire au plan de symétrie, est supérieure à celle de la butée 13, de sorte que, lorsque l'insert tibial 5 et le tenon 2 sont tournés mutuellement, la butée 13 glisse dans la rainure 18 et n'entrave pas cette rotation mutuelle.

Le mode de réalisation décrit ici une seule protubérance disposée opposée à la partie 4 oblongue. On peut cependant également envisager de disposer la protubérance 17 décalée angulairement par rapport à cette direction antéro-postérieure, ou même en prévoir plusieurs, par exemple deux, espacées angulairement l'une de l'autre et coopérant toutes avec la rainure 18.

Une translation médio-latérale est une translation suivant une direction perpendiculaire au plan médial.

## Revendications

1. Prothèse totale d'articulation du genou comprenant, entre une plaque tibiale (1) munie sur sa face intérieure de moyens d'ancrage à une extrémité proximale d'un tibia et une pièce fémorale à condyles munie de moyens d'ancrage à une partie distale d'un fémur, un insert tibial (5) monté glissant sur la plaque tibiale (1) et comportant des surfaces (7) supérieures de forme complémentaire de celles des surfaces extérieures des condyles,
- un tenon (2) étant monté en faisant saillie de la face extérieure de la plaque tibiale (1) et comportant une base (3) et une partie (4) oblongue s'étendant à partir de la base (3) parallèlement à la face extérieure de la plaque tibiale (1) ; et
- l'insert (5) tibial comportant une cavité (9) dans laquelle la partie (4) oblongue est engagée avec possibilité au moins d'une rotation mutuelle de la partie (4) oblongue et de l'insert tibial (5), **caractérisée en ce que** la dimension et la forme de la cavité sont telles qu'elles permettent un déplacement, par rapport à l'insert (5) tibial en translation médio-latérale de la base (3) du tenon (2) dans la cavité (9).

2. Prothèse totale d'articulation du genou suivant la revendication 1, **caractérisée en ce que** le tenon (2) est monté fixe sur la plaque (1) tibiale.

3. Prothèse totale d'articulation du genou suivant la revendication 1 ou 2, **caractérisée en ce que** la partie (4) oblongue est à distance de la plaque (1) tibiale, la cavité (9) comprend une première ouverture (11) latérale ménagée dans la paroi latérale antérieure de l'insert tibial et une deuxième ouverture (12) de fond, la dimension de l'ouverture (11) latérale permettant le passage de la partie (4) oblongue à travers elle et l'ouverture (12) de fond étant délimitée par des bords inférieurs des parois latérales (15, 16) de la cavité (9) et par le bord inférieur du rebord inférieur (13) de la première ouverture (11) latérale, de manière à former un trou de limitation qui limite le déplacement en glissement en translation antéro-postérieur et médio-latéral de la base (3) du tenon (2) par rapport à la plaque (1) tibiale, le rebord inférieur (13) de la première ouverture formant une butée qui coopère avec la partie (4) oblongue de manière à empêcher un détachement par le haut de l'insert tibial de la plaque tibiale.

4. Prothèse totale d'articulation du genou suivant la revendication 3, **caractérisée en ce que** le trou de limitation a la forme d'un triangle isocèle dont les coins sont de forme complémentaire de la forme de la paroi latérale de la base (3) du tenon (2).

5. Prothèse totale d'articulation du genou suivant la revendication 4, **caractérisée en ce que** l'angle du triangle isocèle est compris entre 30° et 50°.

6. Prothèse totale d'articulation du genou suivant l'une des revendications précédentes, **caractérisée en ce que** la surface de la paroi latérale de la base (3) est circulaire.

7. Prothèse totale d'articulation du genou suivant l'une des revendications précédentes, **caractérisée en ce que** les deux parois (15, 16) latérales de la cavité (9) s'étendent du coin formé à leur intersection mutuelle vers le côté antérieur du genou, l'angle du coin formé entre elles étant compris entre 30° et 50°.

8. Prothèse totale d'articulation du genou suivant l'une des revendications 2 à 7, **caractérisée en ce qu'**il est prévu une saillie (17) auxiliaire issue de la base (3) du tenon (2) qui peut pénétrer dans un logement (18) auxiliaire de la cavité (9), le logement (18) étant de forme complémentaire à la forme de la protubérance, le bord inférieur du logement (18) formant une seconde butée (19) coopérant avec la saillie (17) auxiliaire pour empêcher un basculement de l'insert.

9. Prothèse totale d'articulation du genou suivant la revendication 8, **caractérisée en ce que** la saillie (17) auxiliaire coopère avec la seconde butée (19) pour empêcher un basculement de l'insert de l'arrière du genou vers l'avant de celui-ci.

10. Prothèse totale d'articulation du genou suivant l'une des revendications 7 à 10, **caractérisée en ce que** la saillie (17) a la forme d'un demi anneau circulaire issu du sommet de la base (3) et le logement (18) auxiliaire est constitué d'une rainure ménagée dans la partie supérieure de la cavité et dont les dimensions le long des parois (15, 16) de la cavité (9) sont suffisantes pour ne pas bloquer la rotation mutuelle de la partie (4) oblongue et de l'insert (5) tibial.

11. Prothèse totale d'articulation du genou suivant l'une des revendications 7 à 11, **caractérisée en ce que** la saillie (17) est issue de la partie (4) oblongue et s'étend dans une direction opposée à celle dans laquelle s'étend la partie (4) oblongue.

12. Prothèse totale suivant l'une des revendications précédentes, **caractérisée en ce que** la base (3) du tenon (2) peut se déplacer dans la cavité (9) aussi suivant une translation antéro postérieure par rapport à l'insert (5).

13. Insert tibial (5) destiné à être utilisé dans une prothèse totale d'articulation du genou suivant l'une des revendications précédentes comportant une cavité (9) dans laquelle la partie (4) oblongue de la prothèse est engagée avec possibilité au moins d'une rotation mutuelle de la partie (4) oblongue et de l'insert (5) tibial, **caractérisé en ce que** la dimension et la forme de la cavité sont telles qu'elles permettent un déplacement par rapport à l'insert (5) tibial, en translation médio-latérale de la base (3) du tenon (2) de la prothèse dans la cavité (9).

## Patentansprüche

1. Kniegelenk-Totalprothese, die zwischen einer Tibiaplatte (1), welche auf ihrer Innenseite mit Mitteln zur Verankerung an einem proximalen Ende einer Tibia versehen ist, und einem Femurteil mit Kondylen, welches mit Mitteln zur Verankerung an einem distalen Teil eines Femurs versehen ist, einen Tibiaeinsatz (5) umfasst, der auf der Tibiaplatte (1) gleitend angebracht ist und Oberflächen (7) mit einer zu denjenigen der Außenflächen der Kondylen ergänzenden Form aufweist,
- wobei ein Zapfen (2) von der Außenseite der Tibiaplatte (1) vorspringend angebracht ist und eine Basis (3) sowie einen länglichen Teil (4), der von der Basis (3) aus parallel zur Außenseite der Tibiaplatte (1) verläuft, umfasst, und
- wobei der Tibiaeinsatz (5) eine Vertiefung (9) umfasst, in die der längliche Teil (4) mit der Möglichkeit wenigstens eines gegenseitigen Drehens des länglichen Teils (4) und des Tibiaeinsatzes (5) eingesteckt ist, **dadurch gekennzeichnet, dass** die Abmessung und die Form der Vertiefung derart sind, dass sie gegenüber dem Tibiaeinsatz (5) eine medio-laterale Verschiebebewegung der Basis (3) des Zapfens (2) in der Vertiefung (9) ermöglichen.

2. Kniegelenk-Totalprothese nach Anspruch 1, **dadurch gekennzeichnet, dass** der Zapfen (2) an der Tibiaplatte (1) fest angebracht ist.

3. Kniegelenk-Totalprothese nach Anspruch 1 oder 2, **dadurch gekennzeichnet, dass** der längliche Teil (4) im Abstand von der Tibiaplatte (1) ist, die Vertiefung (9) eine erste seitliche Öffnung (11), welche in der vorderen Seitenwand des Tibiaeinsatzes ausgebildet ist, sowie eine zweite bodenseitige Öffnung (12) umfasst, wobei die Abmessung der seitlichen Öffnung (11) den Durchgang des länglichen Teils (4) durch sie hindurch ermöglicht und wobei die bodenseitige Öffnung (12) durch untere Kanten der Seitenwände (15, 16) der Vertiefung (9) und durch die untere Kante des unteren Randes (13) der ersten seitlichen Öffnung (11) begrenzt ist, um ein Begrenzungsloch zu bilden, das die anteroposteriore und medio-laterale Verschiebegleitbewegung der Basis (3) des Zapfens (2) gegenüber der Tibiaplatte (1) begrenzt, wobei der untere Rand (13) der ersten Öffnung einen Anschlag bildet, der mit dem länglichen Teil (4) zusammenwirkt, um ein Lösen von oben her des Tibiaeinsatzes von der Tibiaplatte zu verhindern.

4. Kniegelenk-Totalprothese nach Anspruch 3, **dadurch gekennzeichnet, dass** das Begrenzungsloch die Form eines gleichschenkligen Dreiecks aufweist, dessen Ecken eine die Form der Seitenwand der Basis (3) des Zapfens (2) ergänzende Form haben.

5. Kniegelenk-Totalprothese nach Anspruch 4, **dadurch gekennzeichnet, dass** der Winkel des gleichschenkligen Dreiecks zwischen 30° und 50° beträgt.

6. Kniegelenk-Totalprothese nach einem der vorhergehenden Ansprüche, **dadurch gekennzeichnet, dass** die Oberfläche der Seitenwand der Basis (3) kreisförmig ist.

7. Kniegelenk-Totalprothese nach einem der vorhergehenden Ansprüche, **dadurch gekennzeichnet, dass** die beiden Seitenwände (15, 16) der Vertiefung (9) sich von der an ihrem gegenseitigen Schnittpunkt gebildeten Ecke in Richtung der Vorderseite des Knies erstrecken, wobei der Winkel der zwischen ihnen gebildeten Ecke zwischen 30° und 50° beträgt.

8. Kniegelenk-Totalprothese nach einem der Ansprüche 2 bis 7, **dadurch gekennzeichnet, dass** ein aus der Basis (3) des Zapfens (2) hervorgegangener Hilfsvorsprung (17) vorgesehen ist, der in eine Hilfsaufnahme (18) der Vertiefung (9) eindringen kann, wobei die Aufnahme (18) eine zu der Form des Vorsprungs ergänzende Form aufweist, wobei die untere Kante der Aufnahme (18) einen zweiten Anschlag (19) bildet, der mit dem Hilfsvorsprung (17) zusammenwirkt, um ein Kippen des Einsatzes zu verhindern.

9. Kniegelenk-Totalprothese nach Anspruch 8, **dadurch gekennzeichnet, dass** der Hilfsvorsprung (17) mit dem zweiten Anschlag (19) zusammenwirkt, um ein Kippen des Einsatzes von der Rückseite des Knies zu dessen Vorderseite zu verhindern.

10. Kniegelenk-Totalprothese nach einem der Ansprüche 7 bis 10, **dadurch gekennzeichnet, dass** der Vorsprung (17) die Form eines aus dem Scheitel der Basis (3) hervorgegangenen kreisförmigen Halbrings aufweist und die Hilfsaufnahme (18) von einer Nut gebildet ist, welche in dem oberen Teil der Vertiefung ausgebildet ist und deren Abmessungen entlang der Wände (15, 16) der Vertiefung (9) ausreichend sind, um das gegenseitige Drehen des länglichen Teils (4) und des Tibiaeinsatzes (5) nicht zu blockieren.

11. Kniegelenk-Totalprothese nach einem der Ansprüche 7 bis 11, **dadurch gekennzeichnet, dass** der Vorsprung (17) aus dem länglichen Teil (4) hervorgegangen ist und sich in einer Richtung erstreckt, die zu derjenigen, in die der längliche Teil (4) verläuft, entgegengesetzt ist.

12. Totalprothese nach einem der vorhergehenden Ansprüche, **dadurch gekennzeichnet, dass** die Basis (3) des Zapfens (2) in der Vertiefung (9) gegenüber dem Einsatz (5) auch anteroposterior verschiebebeweglich ist.

13. Tibiaeinsatz (5), der dazu bestimmt ist, bei einer Kniegelenk-Totalprothese nach einem der vorhergehenden Ansprüche verwendet zu werden, umfassend eine Vertiefung (9), in die der längliche Teil (4) der Prothese mit der Möglichkeit wenigstens eines gegenseitigen Drehens des länglichen Teils (4) und des Tibiaeinsatzes (5) eingesteckt ist, **dadurch gekennzeichnet, dass** die Abmessung und die Form der Vertiefung derart sind, dass sie gegenüber dem Tibiaeinsatz (5) eine medio-laterale Verschiebebewegung der Basis (3) des Zapfens (2) der Prothese in der Vertiefung (9) ermöglichen.

## Claims

1. A total knee prosthesis comprising a tibial insert (5) between a tibial plate (1) provided on its inner surface with means for anchoring to a proximal end of a tibia, and a femoral part comprising condyles provided with means for anchoring to a distal part of a femur, the tibial insert (5) being slidably mounted on the tibial plate (1) and comprising upper surfaces (7) complementary in shape with outer surfaces of the condyles,
- a tenon (2) being mounted so as to project from the outer surface of the tibial plate (1) and comprising a base (3) and an oblong part (4) extending from the base (3) parallel to the outer surface of the tibial plate (1); and
- the tibial insert (5) comprising a cavity (9) in which the oblong part (4) engages with possibility at least of rotation of the oblong part (4) relative to the tibial part (5), **characterised in that** the size and shape of the cavity are such that the base (3) of the tenon (2) in the cavity (9) can move relative to the tibial insert (5) in medio-lateral translation.

2. A total knee-joint prosthesis according to claim 1, **characterised in that** the tenon (2) is mounted fixed on the tibial plate (1).

3. A total knee-joint prosthesis according to claim 1 or 2, **characterised in that** the oblong part (4) is at a distance from the tibial plate (1), the cavity (9) comprises a first lateral opening (11) formed in the anterior lateral wall of the tibial insert and a second rear opening (12), the lateral opening (11) being of a size allowing the oblong part (4) to move through it and the rear opening (12) being bounded by bottom edges of the lateral walls (15, 16) of the cavity (9) and by the bottom edge of the bottom rim (13) of the lateral first opening (11), so as to form a hole which limits the sliding motion in antero-posterior or medio-lateral translation of the base (3) of the tenon (2) relative to the tibial plate (1), and the bottom rim (13) of the first opening forms an abutment which co-operates with the oblong part (4) so as to prevent upward detachment of the tibial insert from the tibial plate.

4. A total knee-joint prosthesis according to claim 3, **characterised in that** the hole is in the shape of an isosceles triangle having corners complementary in shape with the lateral wall of the base (3) of the tenon (2).

5. A total knee-joint prosthesis according to claim 4, **characterised in that** the angle of the isosceles triangle is between 30° and 50°.

6. A total knee-joint prosthesis according to any of the preceding claims, **characterised in that** the surface of the lateral wall of the base (3) is circular.

7. A total knee-joint prosthesis according to any of the preceding claims, **characterised in that** the two lateral walls (15, 16) of the cavity (9) extend from the corner formed at their mutual intersection to the anterior side of the knee, the corner angle formed between them being between 30° and 50°.

8. A total knee-joint prosthesis according to any of claims 2 to 7, **characterised in that** an auxiliary projection (17) is provided from the base (3) of the tenon (2) and can be inserted into an auxiliary recess (18) in the cavity (9), the recess (18) being complementary in shape with the protuberance, the bottom edge of the recess (18) forming a second abutment (19) which co-operates with the auxiliary projection (17) so as to prevent the insert from tilting.

9. A total knee-joint prosthesis according to claim 8, **characterised in that** the auxiliary projection (17) co-operates with the second abutment (19) to prevent the insert tilting from the rear of the knee towards the front thereof.

10. A total knee-joint prosthesis according to any of claims 7 to 10, **characterised in that** the projection (17) is in the form of a circular half-ring extending from the top of the base (3) and the auxiliary recess (18) comprises a groove formed in the upper part of the cavity and dimensioned along the walls (15, 16) of the cavity (9) sufficiently so as not to block rotation of the oblong part (4) relative to the tibial insert (5).

11. A total knee-joint prosthesis according to any of claims 7 to 11, **characterised in that** the projection (17) proceeds from the oblong part (4) and extends in the opposite direction to the oblong part (4).

12. Total prosthesis as defined in one of the previous claims, **characterized in that** the base (3) of the tenon (2) can move in the cavity (9) also along an antero posterior translation relative to the insert (5).

13. Tibial insert (5) to be used in a total knee-joint prosthesis as defined in one of the previous claims, comprising a cavity (9) in which the oblong part (4) of the prosthesis engages with possibility at least of rotation of the oblong part (4) relative to the tibial insert (5), **characterized in that** the size and shape of the cavity are such that the base (3) of the tenon (2) of the prosthesis can move, relative to the tibial insert (5), in medio-lateral translation in the cavity (9).
